# EUROPÄISCHE PATENTSCHRIFT

(11) **EP 3 389 792 B1**
(45) Veröffentlichungstag und Bekanntmachung des Hinweises auf die Patenterteilung: **09.09.2020**
(21) Anmeldenummer: 16819855.4
(22) Anmeldetag: 15.12.2016
(51) Int. Cl.: A61Q 5/10, A61K 8/36, A61K 8/20, A61K 8/23, A61K 8/24, A61K 8/34, A61K 8/31, A61K 8/86, A61K 8/37

(54) **VERFAHREN ZUM FÄRBEN VON KERATINISCHEN FASERN**
METHOD FOR COLORING KERATINE FIBERS
PROCEDE DE COLORATION DE FIBRES KERATINE

(30) Priorität: 18.12.2015 DE 102015225897
(43) Veröffentlichungstag der Anmeldung: 24.10.2018
(73) Patentinhaber: Henkel AG & Co. KGaA, 40589 Düsseldorf (DE)
(72) Erfinder: GROSJACQUES, Camille, 20255 Hamburg (DE); WAGNER, Aileen, 21109 Hamburg (DE); HAGENOW, Susanne, 20359 Hamburg (DE); MANNECK, Hartmut, 23858 Barnitz (DE); KLEEN-FEHRES, Astrid, 20457 Hamburg (DE)
(86) Internationale Anmeldenummer: PCT/EP2016/081193
(87) Internationale Veröffentlichungsnummer: WO 2017/102946

(56) Entgegenhaltungen:
- EP-A1- 1 655 056
- WO-A1-2015/153819
- WO-A2-01/97756
- WO-A2-03/068177
- WO-A2-2013/126657

## Beschreibung

Die vorliegende Erfindung liegt auf dem Gebiet der Kosmetik und betrifft Verfahren zum oxidativen Färben von Haaren, wobei Anwendungsmischungen zum Einsatz kommen, die über einen speziellen Mischungsprozess hergestellt werden. Das erfindungsgemässe Verfahren oder die Erfindung ist ausschliesslich, wie in Anspruch 1 definiert, die anfolgenden Teile der Beschreibung sind streng nur in diesem Kontext zu verstehen.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln untereinander die eigentlichen Farbstoffe ausbilden. Oxidationsfärbemittel zeichnen sich durch lang anhaltende Färbeergebnisse aus.

Im Friseurbereich besonders häufig verwendet werden die Level 3 Colorationen. Bei den Level 3 Colorationen handelt es sich um oxidative Färbemittel, die sich durch eine besonders gute Haltbarkeit und eine besonders hohes Grauabdeckungsvermögen auszeichnen.

Erzielt werden können diese gute Haltbarkeit und die gute Grauabdeckung durch einen hohen Ammoniak-Gehalt in den Colorationen, der zu einer starken Quellung des Haares und hierdurch bedingt zu einer hohen Diffusionsrate der Oxidationsfarbstoffvorprodukte in das Haar hinein führt. Bei dunklen Nuancen der Level 3 Colorationen ist zudem der Gehalt an Oxidationsfarbstoffvorprodukten vergleichsweise hoch.

Verbunden mit diesem hohen Ammoniak-Gehalt ist jedoch ebenfalls auch eine hohe Haarschädigung.

Im Heimanwenderbereich kann der Anwender, der diese hohe Haarschädigung nicht bei jeder Färbung nicht in Kauf nehmen möchte, auf Level 2 Produkte ausweichen. Bei den Level 2 Produkten handelt es sich ebenfalls um oxidative Färbemittel, jedoch ist deren Ammoniak-Gehalt geringer, oder aber es werden anstatt Ammoniak alternative, weniger stark quellende Alkalisierungsmittel eingesetzt. Im Heimanwenderbereich sind Level 3 und Level 2 Produkte separat verpackt und werden als getrennte Produkte vertrieben, so dass der Anwender sich entweder ein Level 3 oder ein Level 2 Produkt aussuchen und applizieren kann.

Im Friseurbereich stellt der Friseur seinem Kunden eine viel umfangreichere Nuancenpalette zur Verfügung. Somit umfasst eine komplette Level 3 Färbeserie eine Palette verschiedenster Farbcremes, welche jeweils kurz vor der Anwendung mit der üblichen Level 3 Oxidationsmittelzubereitung vermischt werden. Aus Kapazitäts- und Lagergründen wird der Friseur es daher vermeiden, sowohl eine vollständige Nuancenpalette für Level 3 Produkte und für Level 2 Produkte vorrätig zu halten.

Es war daher eine erste Aufgabe der vorliegenden Erfindung, dem Friseur einen flexibel und leicht anwendbare Methode zur Verfügung zu stellen, die es dem Friseur erlaubt, aus einem Level 3 Färbeprodukt ein Level 2 Produkt herzustellen.

Darüber hinaus ist der Bereich des Haares von der Wurzel zur Spitze verschieden stark geschädigt. Haare im Bereich des Ansatzes sind gerade frisch nachgewachsen und wurden noch keinen Witterungseinflüssen oder chemischen Einflüssen ausgesetzt. Die Haare im Bereich der Spitzen hingegen sind die ältesten Teile des Haares und weisen daher die stärkste Schädigung auf.

In geschädigtem Haar ist die Cuticula, die Schuppenschicht des Haares, in einem mehr oder weniger starken Ausmaß zerstört. Dies führt dazu, dass auf geschädigtem Haar generell ein stärkerer Farbaufzug stattfindet. Wenn Ansatz und Spitzen mit demselben Färbemittel gefärbt werden, besteht bei stärker geschädigtem Haar daher immer die Gefahr eines ungleichmäßigen Farbergebnisses.

Es war eine weitere Aufgabe der vorliegenden Erfindung, dem Friseur ein System zur Verfügung zu stellen, mit dem er nach der Beurteilung des Schädigungsgrades des zu färbenden Haares die Farbstoffkonzentration im Haarfärbemittel einfach, gezielt und genau reproduzierbar herabsetzen kann.

Sowohl die Herstellung eines Level 2 Färbe-Produktes aus einem Level 3 Produkt als auch die Verminderung der Farbstoffkonzentration für die Anwendung auf bestimmten Haarpartien kann prinzipiell durch eine "Verdünnung" des oxidativen Level 3 Produktes erzielt werden. Hierzu sind aus dem Stand der Technik bereits verschiedene Möglichkeiten bekannt. So wie z.B. in der WO 2013/126657 A2 beschrieben.

Eine Möglichkeit zur Verdünnung ist beispielsweise das Vermischen des anwendungsbereiten oxidativen Färbemittels mit einem Conditioner. Da Conditioner oft auf einen leicht sauren pH-Wert eingestellt sind, kann der pH-Wert des Färbemittels so gesenkt und das Ausmaß der Haarquellung vermindert werden. Bedingt durch die im Conditioner enthaltenen Pflegesubstanzen (bestimmte Polymere, Silikone, ionische Tenside etc.) kann jedoch durch die Verdünnung mit dem Conditioner eine Farbverschiebung auftreten, so dass das Farbresultat nicht mehr mit der gewünschten Nuance übereinstimmt.

Eine weitere Möglichkeit zur Verdünnung besteht im Vermischen des anwendungsbereiten oxidativen Färbemittels mit einem Shampoo. Da Shampoos größere Mengen an Reinigungstensiden enthalten, kann während der Anwendung die Schaumentwicklung zu stark ausfallen, so dass die vollständige Benetzung des Haares mit dem verdünnten Färbemittel nicht mehr gewährleistet ist. Falls das Shampoo nicht ausreichend sauer eingestellt ist, werden zudem zu große Shampoo-Mengen zur Verdünnung des Färbemittels notwendig.

Dasselbe Problem tritt auch auf, wenn das anwendungsbereite Mittel mit reinem Wasser verdünnt wird. Da Wasser keine Säuren enthält, werden zur Absenkung des pH-Wertes des Färbemittels sehr hohe Wassermengen benötigt. Schließlich macht auch der Viskositätsunterschied zwischen dem Wasser und der angedickten Färbecreme ein schnelles Vermischen unmöglich.

Es hat sich nun überraschenderweise herausgestellt, dass das Färbemittel flexibel, schnell und auf komfortable Weise ohne die zuvor beschriebenen Nachteile verdünnt werden kann, wenn der Friseur einem Verfahren folgt, bei dem zuerst eine Farbcreme, welche die Oxidationsfarbstoffvorprodukte enthält, mit einem speziellen "Farbverdünner" vermischt und aus diesem beiden Komponenten eine erste homogene Mischung hergestellt wird. Diese Mischung wird dann in einem nächsten Schritt mit der Oxidationsmittelzubereitung vermischt, und diese zweite Mischung wird dann auf die Haare appliziert.

Gegenstand der vorliegenden Erfindung ist ausschliesslich ein Verfahren zum Färben von menschlichen Haaren, umfassend die folgenden Schritte in der angegebenen Reihenfolge
A) Vermischen einer ersten Komponente (K1) mit einer zweiten Komponente (K2) unter Erhalt einer ersten Mischung (M1),
B) Vermischen der Mischung (M1) mit einer dritten Komponente (K3) unter Erhalt einer zweiten Mischung (M2),
C) Applizieren der Mischung (M2) auf die Haare,
D) Einwirkenlassen der Mischung (M2) für einen Zeitraum von 30 Sekunden bis 45 Minuten,
E) Ausspülen der Mischung (M2) aus den Haaren,
   wobei
   - die erste Komponente (K1) eine Färbezubereitung ist, die mindestens ein Oxidationsfarbstoffvorprodukt enthält, und
   - die zweite Komponente (K2) ein Farbverdünner ist, der selbst keine Oxidationsfarbstoffvorprodukte und kein Oxidationsmittel enthält, und - bezogen auf das Gesamtgewicht der zweiten Komponente (K2) - einen oder mehrere Fettbestandteile in einer Gesamtmenge von 5,0 bis 70,0 Gew.-% und eine oder mehrere Säuren in einer Gesamtmenge von 0.5 bis 15.0 Gew.-% enthält, und
   - die dritte Komponente (K3) eine Oxidationsmittelzubereitung ist, die mindestens ein Oxidationsmittel enthält.

Bei dem erfindungsgemäßen Verfahren handelt es sich um ein Verfahren zum Färben von menschlichen Haaren, bei dem die drei Komponenten (K1), (K2) und (K3) sukzessive miteinander vermischt werden. Bei allen drei Komponenten (K1), (K2) und (K3) handelt es sich um kosmetische Mittel, welche alle wesentlichen Inhaltsstoffe jeweils in einem kosmetischen Träger enthalten.

In einem ersten Schritt A) wird eine erste Komponente (K1) mit einer zweiten Komponente (K2) vermischt. Hierbei handelt es sich bei der ersten Komponente (K1) um eine Färbezubereitung, die in einem kosmetischen Träger mindestens ein Oxidationsfarbstoffvorprodukt enthält. Die erste Komponente (K1) enthält mindestens ein Oxidationsfarbstoffvorprodukt vom Entwicklertyp und mindestens ein Oxidationsfarbstoffvorprodukt vom Kupplertyp.

Bei der zweiten Komponenten (K2) handelt es sich um einen "Farbverdünner", wobei die zweite Komponente dadurch gekennzeichnet ist, dass sie selbst keine Oxidationsfarbstoffvorprodukte, keine direktziehenden Farbstoffe und auch kein Oxidationsmittel (wie beispielsweise Wasserstoffperoxid und Persulfatsalze) enthält.

Unter dem Begriff "Farbverdünner" wird im Sinne der vorliegenden Verbindung eine separat verpackte Zubereitung (K2) verstanden, die mit der Färbezubereitung (K1) vermischt werden und auf diese Weise den Gehalt des in der Komponente (K1) enthalten Ammoniaks und der Oxidationsfarbstoffvorprodukte gezielt absenken soll.

Mit anderen Worten handelt es sich bei der Komponente (K2) weder um eine Färbezubereitung noch um eine Oxidationsmittelzubereitung.

Die erste Komponente (K1) und die zweite Komponente (K2) können beispielsweise durch Rühren oder Verschütteln miteinander vermischt werden, wodurch die erste Mischung (M1) entsteht. Die Mischung (M1) wird erfindungsgemäß nicht auf die Haare appliziert.

Als besonders vorteilhaft hat sich herausgestellt, dass die Verdünnung des Färbemittels, d.h. die Herstellung der ersten Mischung (M1) aus Färbezubereitung (K1) und Farbverdünner (K2), durchgeführt werden konnte, ohne dass die durch die Zugabe des Oxidationsmittels bereits die Farbstoffbildung aus den Oxidationsfarbstoffvorprodukten initiiert wurde. Auf diese Weise konnte ein gleichmäßigeres und reproduzierbareres Farbergebnis erzielt werden.

Aus diesem Grund ist die Herstellung dieser ersten Mischung (oder Vormischung) (M1) aus (K1) und (K2) in Schritt A) ein ganz zentraler und wesentlicher Schritt des erfindungsgemäßen Verfahrens.

Wurde hingegen zuerst ein übliches oxidatives Färbemittel durch Vermischen der Komponenten (K1) und (K3) hergestellt und dieses danach mit der Komponente (K2) verdünnt, so liefen der Farbstoffbildungsprozess und die Verdünnung parallel ab. Bei diesem - nicht erfindungsgemäßen - Verfahren wurde die Intensität der finalen Haarfärbung somit wesentlich von der Dauer mitbeeinflusst, die für das Untermischen der Komponente (K2) benötigt wurde. Die Färberesultate, die über dieses nicht erfindungsgemäße Verfahren erzielt wurden, waren somit weniger reproduzierbar.

Wenn die Komponenten (K1) und (K2) vollständig miteinander vermischt sind, d.h. wenn eine homogene Mischung (M1) vorliegt, wird diese Mischung im nächsten Schritt B) wiederum mit der dritten Komponente (K3) vermischt.

Die dritte Komponente (K3) ist eine Oxidationsmittelzubereitung, welche mindestens ein Oxidationsmittel enthält. Bei diesem Oxidationsmittel handelt es sich ganz besonders bevorzugt um Wasserstoffperoxid.

Die erste Mischung (M1) kann mit der dritten Komponente (K3) wiederum durch Rühren oder Verschütteln vermischt werden. Bei diesem zweiten Mischvorgang in Schritt B) entsteht dann die zweite Mischung (M2). Bei der zweiten Mischung handelt es sich um das anwendungsbereite oxidative Färbemittel, welches auf die Haare appliziert wird.

Das Auftragen der zweiten Mischung (M2) auf die Haare erfolgt erfindungsgemäß im dritten Schritt C), wobei die Mischung (M2) entweder auf den gesamten Bereich der zu färbenden Haare oder nur auf bestimmte Haarpartien (wie beispielsweise den Haaransatz oder die Haarlängen/Spitzen) aufgetragen werden kann.

Nach dem Auftragen wird die Mischung (M2) dann in Schritt D) für einen Zeitraum von 30 Sekunden bis 45 Minuten auf die Haare einwirken gelassen. Hierbei ist es möglich und erfindungsgemäß, die Mischung (M2) für einen bestimmten Zeitraum auf allen Partien des Haares zu belassen. In einer weiteren Ausführungsform ist es jedoch ebenfalls möglich, die Einwirkdauer für bestimmte Haarpartien verschieden zu wählen, so dass beispielsweise die Einwirkdauer im Bereich das Haaransatzes länger ist als die Einwirkdauer im Bereich der geschädigten Spitzen.

Nach dem Einwirkenlassen wird die Mischung (M2) dann in Schritt E) wieder aus den Haaren ausgespült. Das Ausspülen kann entweder nur mit Wasser oder aber unter Zuhilfenahme eines Shampoos erfolgen.

Die Schritte A) bis E) sind die Schritte eines einzigen Färbeverfahrens, d.h. alle Schritte werden erfindungsgemäß während eines Färbevorgangs, d.h. innerhalb eines Zeitraumes von maximal 6 Stunden, bevorzugt innerhalb eines Zeitraums von maximal 3 Stunden, durchgeführt.

Im erfindungsgemäßen Verfahren ist auch die Reihenfolge der Schritte festgelegt und findet in der Reihenfolge A), gefolgt von B), gefolgt von C), gefolgt von D) gefolgt von E) statt.

Eine besonders bevorzugte Ausführungsform ist somit ein Verfahren zum Färben von menschlichen Haaren, umfassend die folgenden Schritte in der angegebenen Reihenfolge
A) Vermischen einer ersten Komponente (K1) mit einer zweiten Komponente (K2) unter Erhalt einer ersten Mischung (M1),
B) Vermischen der Mischung (M1) mit einer dritten Komponente (K3) unter Erhalt einer zweiten Mischung (M2),
C) Applizieren der Mischung (M2) auf die Haare,
D) Einwirkenlassen der Mischung (M2) für einen Zeitraum von 30 Sekunden bis 45 Minuten,
E) Ausspülen der Mischung (M2) aus den Haaren,
   wobei
   - die erste Komponente (K1) eine Färbezubereitung ist, die in einem kosmetischen Träger mindestens ein Oxidationsfarbstoffvorprodukt vom Entwicklertyp und ein mindestens ein Oxidationsfarbstoffvorprodukt vom Kupplertyp enthält,
   - die zweite Komponente (K2) ein "Farbverdünner" ist, der selbst keine Oxidationsfarbstoffvorprodukte und kein Oxidationsmittel enthält, und - bezogen auf das Gesamtgewicht der zweiten Komponente (K2) - einen oder mehrere Fettbestandteile in einer Gesamtmenge von 5,0 bis 70,0 Gew.-% und eine oder mehrere Säuren in einer Gesamtmenge von 0.5 bis 15.0 Gew.-% enthält, und
   - die dritte Komponente (K3) eine Oxidationsmittelzubereitung ist, die in einem kosmetischen Träger Wasserstoffperoxid enthält.

Wesentlich für eine optimale Verdünnung der Färbezubereitung (K1) und für ein späteres homogenes und reproduzierbares Farbresultat ist die Beschaffenheit des Farbverdünners, d.h. der zweiten Komponente (K2).

Um eine schnelle und gute Vermischbarkeit der Komponenten (K1) und (K2) zu gewährleisten, ist die Komponente (K2) besonders bevorzugt mit einem oder mehreren Fettbestandteilen verdickt und liegt somit in Form einer Emulsion vor.

In einer besonders bevorzugten Ausführungsform ist das erfindungsgemäße Verfahren zum Färben von menschlichen Haaren dadurch gekennzeichnet, dass die zweite Komponente (K2) - bezogen auf das Gesamtgewicht der zweiten Komponente (K2) - einen oder mehrere Fettbestandteile in einer Gesamtmenge von 5,0 bis 70,0 Gew.-%, bevorzugt 10,0 bis 65,0 Gew.-%, weiter bevorzugt von 20,0 bis 60,0 Gew.-% und ganz besonders bevorzugt von 40,0 bis 60,0 Gew.-% enthält.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 8 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Als bevorzugte Fettbestandteile werden in diesem Zusammenhang die Bestandteile aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den C₁₂-C₃₀-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenoylalkohol), Gadoleylalkohol ((9Z)-Eicos-9-en-1-ol), Arachidonalkohol ((5Z,8Z,11Z,14Z)-Eicosa-5,8,11,14-tetraen-1-ol), Erucylalkohol ((13Z)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Unter einem C₁₂-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₁₂-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₁₂-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₁₂-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)-Tetracos-15-ensäure].

Unter einem C₁₂-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Geeignete Paraffinöle sind insbesondere flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Bevorzugte Fettbestandteile sind ausgewählt aus der Gruppe der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe. Die C₁₂-C₃₀-Fettalkohole und/oder die Kohlenwasserstoffe sind bevorzugte Fettbestandteile. Ganz besonders bevorzugte Fettbestandteile sind die C₁₂-C₃₀-Fettalkohole.

Die Komponente (K2) enthält besonders bevorzugt einen oder mehrere Fettbestandteile in einer Gesamtmenge von 5,0 bis 70,0 Gew.-%, bevorzugt 10,0 bis 65,0 Gew.-%, weiter bevorzugt von 20,0 bis 60,0 Gew.-% und ganz besonders bevorzugt von 40,0 bis 60,0 Gew.-%. Hierbei sind alle Mengenangaben in Gew.-% auf die Gesamtmenge aller in der Komponente (K2) enthaltenen Fettbestandteile bezogen, die zum Gesamtgewicht der Komponente (K2) in Relation gesetzt wird.

Im Hinblick auf eine gute und reproduzierbare Verdünnung hat es sich als ganz besonders bevorzugt erwiesen, wenn die zweite Komponente (K2) - bezogen auf das Gesamtgewicht der zweiten Komponente (K2) - einen oder mehrere Kohlenwasserstoffe in einer Gesamtmenge von 20,0 bis 65,0 Gew.-%, bevorzugt von 25,0 bis 60,0 Gew.-%, weiter bevorzugt von 35,0 bis 60,0 Gew.-% und besonders bevorzugt von 40,0 bis 55,0 Gew.-% enthält.

In einer besonders bevorzugten Ausführungsform ist das Verfahren zum Färben von menschlichen Haaren dadurch gekennzeichnet, dass die zweite Komponente (K2) - bezogen auf das Gesamtgewicht der zweiten Komponente (K2) - einen oder mehrere Kohlenwasserstoffe in einer Gesamtmenge von 20,0 bis 65,0 Gew.-%, bevorzugt von 25,0 bis 60,0 Gew.-%, weiter bevorzugt von 35,0 bis 60,0 Gew.-% und besonders bevorzugt von 40,0 bis 55,0 Gew.-% enthält.

Ziel des Vermischens der Farbcreme (K1) mit dem Verdünner (K2) ist eine gezielte Reduktion des pH-Wertes und damit verbunden eine Verminderung der Quellung des Haares während der Anwendung. Die Mischung (M1), die durch Vermischen von (K1) und (K2) erzielt wird, besitzt somit einen niedrigeren pH-Wert als die Farbcreme (K1). Als Folge hiervon besitzt auch die Mischung (M2), die durch Vermischen von (M1) mit (K3) erzielt wird, einen niedrigeren pH-Wert als die Mischung aus (K1) und (K3) (d.h. (M2) besitzt einen niedrigeren pH-Wert als das unverdünnte oxidative Färbemittel). Auf diese Weise soll der Friseur in die Lage versetzt werden, des Haar des Kunden - das ggf. nach wiederholten Färbebehandlungen stark geschädigt ist - ohne Farbverschiebung in der gewohnten Nuance zu färben, hierbei jedoch zusätzliche Haarschädigungen nach Möglichkeit zu vermeiden.

Zur Reduktion des pH-Wertes enthält die zweite Komponente (K2) mindestens eine Säure. Eine Reduktion des pH-Wertes ohne wahrnehmbare Farbverschiebung (zwischen verdünntem und nicht verdünntem Färbemittel) ist möglich, wenn die Komponente (K2) mindestens eine Säure aus der Gruppe aus Milchsäure, Zitronensäure, Äpfelsäure, Weinsäure, Maleinsäure, Bernsteinsäure, Oxalsäure, Ascorbinsäure, Schwefelsäure, Salzsäure und/oder Phosphorsäure enthält.

In einer besonders bevorzugten Ausführungsform ist das Verfahren zum Färben von menschlichen Haaren dadurch gekennzeichnet, dass die zweite Komponente (K2) mindestens eine Säure aus der Gruppe aus Milchsäure, Zitronensäure, Äpfelsäure, Weinsäure, Maleinsäure, Bernsteinsäure, Oxalsäure Ascorbinsäure, Schwefelsäure, Salzsäure und/oder Phosphorsäure enthält.

Als ganz besonders gut geeignet hat sich in diesem Zusammenhang der Zusatz von Milchsäure erwiesen.

In einer ganz besonders bevorzugten Ausführungsform ist das Verfahren zum Färben von menschlichen Haaren, dadurch gekennzeichnet, dass die zweite Komponente (K2) Milchsäure, enthält.

Um eine effektive Absenkung des pH-Wertes gewährleisten zu können, werden die Säure bzw. die Säuren zu der Komponente (K2) bevorzugt in einer Gesamtmenge von 0,5 bis 15,0 Gew.-%, bevorzugt 0,5 bis 8,0 Gew.-% weiter bevorzugt 1,5 bis 6,0 Gew.-% und ganz besonders bevorzugt von 2,5 bis 5,5 Gew.-% hinzugefügt. Hierbei sind alle Angaben in Gew.-% auf die Gesamtmenge aller in der Komponente (K2) enthaltenen Säuren bezogen, die zum Gesamtgewicht der Komponente (K2) in Relation gesetzt wird.

In einer besonders bevorzugten Ausführungsform ist das Verfahren zum Färben von menschlichen Haaren, dadurch gekennzeichnet, dass die zweite Komponente (K2) - bezogen auf das Gesamtgewicht der zweiten Komponente - eine oder mehrere Säuren in einer Gesamtmenge von 0,5 bis 15,0 Gew.-%, bevorzugt 0,5 bis 8,0 Gew.-% weiter bevorzugt 1,5 bis 6,0 Gew.-% und ganz besonders bevorzugt von 2,5 bis 5,5 Gew.-% enthält.

In einer besonders bevorzugten Ausführungsform ist das Verfahren zum Färben von menschlichen Haaren, dadurch gekennzeichnet, dass die zweite Komponente (K2) - bezogen auf das Gesamtgewicht der zweiten Komponente - 2,5 bis 10,0 Gew.-% Milchsäure enthält.

In einer besonders bevorzugten Ausführungsform ist das Verfahren zum Färben von menschlichen Haaren, dadurch gekennzeichnet, dass die zweite Komponente (K2) 3,0 bis 8,0 Gew.-% Milchsäure enthält.

In einer besonders bevorzugten Ausführungsform ist das Verfahren zum Färben von menschlichen Haaren, dadurch gekennzeichnet, dass die zweite Komponente (K2) 3,5 bis 6,0 Gew.-% Milchsäure enthält.

In einer besonders bevorzugten Ausführungsform ist das Verfahren zum Färben von menschlichen Haaren, dadurch gekennzeichnet, dass die zweite Komponente (K2) 3,8 bis 5,5 Gew.-% Milchsäure enthält.

In einer besonders bevorzugten Ausführungsform ist das Verfahren zum Färben von menschlichen Haaren, dadurch gekennzeichnet, dass die zweite Komponente (K2) 0,5 bis 15,0 Gew.-% Zitronensäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Zitronensäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Zitronensäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Zitronensäure enthält.

In einer besonders bevorzugten Ausführungsform ist das Verfahren zum Färben von menschlichen Haaren, dadurch gekennzeichnet, dass die zweite Komponente (K2) 0,5 bis 15,0 Gew.-% Äpfelsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Äpfelsäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Äpfelsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Äpfelsäure enthält.

In einer besonders bevorzugten Ausführungsform ist das Verfahren zum Färben von menschlichen Haaren, dadurch gekennzeichnet, dass die zweite Komponente (K2) 0,5 bis 15,0 Gew.-% Weinsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Weinsäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Weinsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Weinsäure enthält.

In einer besonders bevorzugten Ausführungsform ist das Verfahren zum Färben von menschlichen Haaren, dadurch gekennzeichnet, dass die zweite Komponente (K2) 0,5 bis 15,0 Gew.-% Maleinsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Maleinsäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Maleinsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Maleinsäure enthält.

In einer besonders bevorzugten Ausführungsform ist das Verfahren zum Färben von menschlichen Haaren, dadurch gekennzeichnet, dass die zweite Komponente (K2) 0,5 bis 10,0 Gew.-% Bernsteinsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Bernsteinsäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Bernsteinsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Bernsteinsäure enthält.

In einer besonders bevorzugten Ausführungsform ist das Verfahren zum Färben von menschlichen Haaren, dadurch gekennzeichnet, dass die zweite Komponente (K2) 0,5 bis 10,0 Gew.-% Oxalsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Oxalsäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Oxalsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Oxalsäure enthält.

In einer besonders bevorzugten Ausführungsform ist das Verfahren zum Färben von menschlichen Haaren, dadurch gekennzeichnet, dass die zweite Komponente (K2) 0,5 bis 15,0 Gew.-% Ascorbinsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Ascorbinsäure enthält.

In einer besonders bevorzugten Ausführungsform ist das Verfahren zum Färben von menschlichen Haaren, dadurch gekennzeichnet, dass die zweite Komponente (K2) 1,5 bis 6,0 Gew.-% Ascorbinsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Ascorbinsäure enthält.

In einer besonders bevorzugten Ausführungsform ist das Verfahren zum Färben von menschlichen Haaren, dadurch gekennzeichnet, dass die zweite Komponente (K2) 0,5 bis 15,0 Gew.-% Phosphorsäure enthält.

In einer besonders bevorzugten Ausführungsform ist das Verfahren zum Färben von menschlichen Haaren, dadurch gekennzeichnet, dass die zweite Komponente (K2) 0,5 bis 8,0 Gew.-% Phosphorsäure enthält.

In einer besonders bevorzugten Ausführungsform ist das Verfahren zum Färben von menschlichen Haaren, dadurch gekennzeichnet, dass die zweite Komponente (K2) 0,5 bis 6,0 Gew.-% Phosphorsäure enthält.

In einer besonders bevorzugten Ausführungsform ist das Verfahren zum Färben von menschlichen Haaren, dadurch gekennzeichnet, dass die zweite Komponente (K2) 2,5 bis 5,5 Gew.-% Phosphorsäure enthält.

In einer weiteren ganz besonders Ausführungsform ist die Komponente (K2) wässrig oder wasserhaltig. Bedingt durch den Gehalt an einer oder mehreren Säuren ist auch der pH-Wert der Komponente (K2) sauer eingestellt und liegt bevorzugt bei einem Wert von 0,5 bis 5,5, bevorzugt von 1,0 bis 4,5, weiter bevorzugt von 1,0 bis 3,0 und ganz besonders bevorzugt von 1,0 bis 2,5.

In einer besonders bevorzugten Ausführungsform ist das Verfahren zum Färben von menschlichen Haaren, dadurch gekennzeichnet, dass die zweite Komponente (K2) wässrig ist und einen pH-Wert von 0,5 bis 5,5, bevorzugt von 1,0 bis 4,5, weiter bevorzugt von 1,0 bis 3,5 und ganz besonders bevorzugt von 1,0 bis 2,5 besitzt.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.

Wie bereits zuvor beschrieben handelt es sich bei der Komponente (K2) ganz besonders bevorzugt um eine Emulsion mit einem verhältnismäßig hohen Gehalt an Fettbestandteilen. Bevorzugt ist der Wassergehalt der Komponente (K2) an diesen Gehalt an Fettbestandteilen angepasst und liegt bei 20,0 bis 70,0 Gew.-%, bevorzugt von 24,0 bis 60,0 Gew.-%, weiter bevorzugt von 24,0 bis 50,0 Gew.-% und ganz besonders bevorzugt von 28,0 bis 38,0 Gew.-%. Die Angaben in Gew.-% sind auch hier wieder auf das Gewicht an Wasser in der Komponente (K2) bezogen, das zum Gesamtgewicht der Komponente (K2) in Relation gesetzt wird.

In einer besonders bevorzugten Ausführungsform ist das Verfahren zum Färben von menschlichen Haaren dadurch gekennzeichnet, dass die zweite Komponente (K2) - bezogen auf das Gesamtgewicht der Komponente (K2) - einen Wassergehalt von 20,0 bis 70,0 Gew.-%, bevorzugt von 24,0 bis 60,0 Gew.-%, weiter bevorzugt von 24,0 bis 50,0 Gew.-% und ganz besonders bevorzugt von 28,0 bis 38,0 Gew.-% besitzt.

Um zu gewährleisten, dass die Komponenten (K2) in Form einer stabilen Emulsion vorliegt, enthält die Komponente (K2) bevorzugt ein oder mehrere Tenside. Der Zusatz zu bestimmten ionischen Tensiden zur Komponente (K2) kann unter Umständen im Vergleich zum unverdünnten Färbemittel eine Färbemittel eine Farbverschiebung hervorrufen, daher werden der Komponente (K2) ganz besonders bevorzugt ein oder mehrere nichtionische Tenside zugesetzt. Nichtionische Tenside können auch als nichtionische Emulgatoren bezeichnet werden.

In einer besonders bevorzugten Ausführungsform ist das Verfahren zum Färben von menschlichen Haaren, dadurch gekennzeichnet, dass die zweite Komponente (K2) eine oder mehrere nichtionische Tenside enthält.

Bei einem nichtionischen Tensid handelt es sich um ein Tensid, das keine Ladung bzw.. keine Ladungen trägt. Mit anderen Worten enthält ein nichtionisches Tensid keine dissoziierbaren funktionellen Gruppen und kann sich in Wasser daher nicht in Ionen auftrennen. Nichtionische Tenside sind aus einem unpolaren Teil, bevorzugt einer Kohlenwasserstoffkette (Alkylkette) mit mindestens 8 Kohlenstoffatomen, und einem polaren Teil aufgebaut. Als polarer Teil kann im nichtionischen Tensid beispielsweise eine Polyethylenglycol-Einheit oder eine (Mono- oder Poly)-Saccharid-Einheit enthalten sein.

Fettalkohole (d.h. C₈-C₃₀-Alkanole) mit einer Fettkette und nur einer Hydroxygruppe besitzen eine sehr schlechte Löslichkeit in Wasser und keinen ausreichend polaren Molekülteil. Daher werden Fettalkohole im Sinne der vorliegenden Erfindung als Fettkomponenten und explizit nicht als nichtionische Tenside betrachtet.

Auch die Monoester und Diester von Fettalkoholen (d.h. C₈-C₃₀-Alkanolen) und Ethylenglycol werden als Fettstoffe und explizit nicht als nichtionische Tenside betrachtet.

Auch die Monoester, Diester und Triester von Fettalkoholen (d.H. C₈-C₃₀-Alkanolen) und Glycerin werden als Fettstoffe und explizit nicht als nichtionische Tenside betrachtet.

Geeignete nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 5 bis 50 Mol Ethylenoxid und/oder 5 bis 50 Mol Propylenoxid an lineare und verzweigte Fettalkohole mit 8 bis 30 C-Atomen, wie beispielsweise Lauryl-, Myristyl-, Cetyl-, aber auch Stearyl-, Isostearyl- und Oleylalkohol, an Fettsäuren mit 8 bis 30 C-Atomen und an Alkylphenole mit 8 bis 15 C-Atomen in der Alkylgruppe,
- mit einem Methyl- oder C₂-C₆-Alkylrest endgruppenverschlossene Anlagerungsprodukte von 5 bis 50 Mol Ethylenoxid und/oder 5 bis 50 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- Polyglycerinester und alkoxylierte Polyglycerinester, wie beispielsweise Poly(3)glycerin-diisostearat (Handelsprodukt: Lameform®TGI (Henkel)) und Poly(2)glycerinpolyhydroxy-stearat (Handelsprodukt: Dehymuls®PGPH (Henkel)).
- Polyolfettsäureester, wie beispielsweise das Handelsprodukt Hydagen® HSP (Cognis) oder Sovermol-Typen (Cognis),
- höher alkoxylierte, bevorzugt propoxylierte und insbesondere ethoxylierte, Mono-, Di- und Triglyceride, wie beispielsweise Glycerinmonolaurat + 20 Ethylenoxid und Glycerinmonostearat + 20 Ethylenoxid,
- Aminoxide,
- Hydroxymischether,
- Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO),
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylphenole und Alkylphenolalkoxylate mit 6 bis 21, insbesondere 6 bis 15 Kohlenstoffatomen in der Alkylkette und 1 bis 30 Ethylenoxid- und/oder Propylenoxid-Einheiten. Bevorzugte Vertreter dieser Klasse sind beispielsweise Nonylphenol + 9 EO und Octylphenol + 8 EO;
- Alkylpolyglykoside entsprechend der allgemeinen Formel RO-(Z)ₓ, wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Als besonders bevorzugte nichtionische Tenside haben sich insbesondere ethoxylierte Fettalkohole sowie C₈-C₂₂-Alkylmono- und oligoglycoside herausgestellt.

In einer besonders bevorzugten Ausführungsform ist das Verfahren zum Färben von menschlichen Haaren dadurch gekennzeichnet, dass die zweite Komponente (K2) einen oder mehrere ethoxylierte Fettalkohole der Formel (I) enthält, 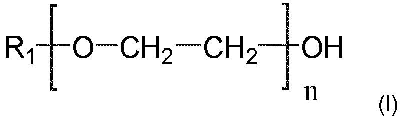 worin
- R1: für eine gesättigte oder ungesättigte, unverzweigte oder verzweigte C₈-C₃₀-Alkylgruppe, bevorzugt für eine gesättigte, unverzweigte C₁₆- oder C₁₈- Alkylgruppe, steht und
- n: für eine ganze Zahl von 10 bis 120, bevorzugt für eine ganze Zahl von 10 bis 80, weiter bevorzugt für eine ganze Zahl von 10 bis 50 und besonders bevorzugt für eine ganze Zahl von 10 bis 30 steht.

In einer besonders bevorzugten Ausführungsform ist das Verfahren zum Färben von menschlichen Haaren auch dadurch gekennzeichnet, dass die zweite Komponente (K2) ein oder mehrere Alkylmono- oder polyglucoside der Formel (II) enthält, 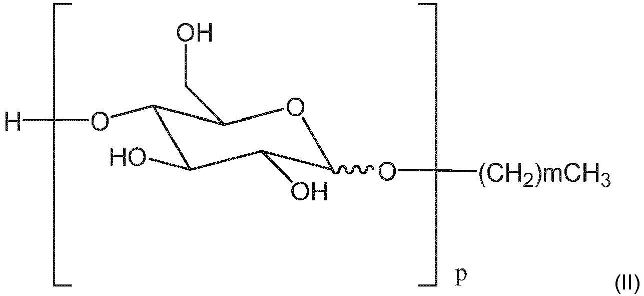 enthält, worin
- m für eine ganze Zahl 7 bis 21, bevorzugt von 9 bis 19, weiter bevorzugt von 9 bis 17 und ganz besonders bevorzugt von 11 bis 15 steht und
- p für eine ganze Zahl von 1 bis 4, bevorzugt von 1 bis 3 und besonders bevorzugt von 1 bis 2 steht.

Das oder die nichtionischen Tenside können in der Komponente (K2) - bezogen auf das Gesamtgewicht der Komponente (K2) - in einer Gesamtmenge von 0,5 bis 9,0 Gew.-%, bevorzugt von 1,5 bis 7,5 Gew.-%, und ganz besonders bevorzugt von 3,5 bis 6,5 Gew.-% enthalten sein.

Wie bereits zuvor beschrieben kann der Zusatz von ionischen oder geladenen Tensiden zum Farbverdünner (K2), wenn dieser zur Verdünnung des aus (K1) und (K3) bestehenden oxidativen Färbemittels eingesetzt wird, im Vergleich zum unverdünnten Färbemittel (d.h. im Vergleich zur Mischung aus (K1) und (K3) allein) zu einer unerwünschten Farbverschiebung führen. Auch der Zusatz von bestimmten Silikonen und Polymeren zur Komponente (K2) kann unter Umständen entsprechende unerwünschte Effekte haben. Aus diesem Grund wird die Komponente (K2) besonders bevorzugt so konfektioniert, dass sie im Wesentlichen aus Fettbestandteilen, Säuren nichtionischen Tensiden und Wasser besteht. Aus diesem Grund ist es besonders bevorzugt, wenn das Gesamtgewicht aus Fettbestandteilen Säuren, nichtionischen Tenside und Wasser mindestens 95,0 Gew.-%, bevorzugt mindestens 97,0 Gew.-%, weiter bevorzugt zu mindestens 99,0 Gew.-% und ganz besonders bevorzugt 100,0 Gew.-% des Gesamtgewichts der Komponente (K2) ausmacht.

In einer weiteren ganz besonders bevorzugten Ausführungsform ist das Verfahren zum Färben von menschlichen Haaren, dadurch gekennzeichnet, dass die zweite Komponente (K2) einen oder mehrere Fettbestandteile und eine oder mehrere Säuren und ein oder mehrere nichtionische Tenside und Wasser enthält, wobei sich - bezogen auf das Gesamtgewicht der zweiten Komponente (K2) - die Mengen aus Fettbestandteilen, Säuren, nichtionischen Tensiden und Wasser zu mindestens 95,0 Gew.-%, bevorzugt mindestens 97,0 Gew.-%, weiter bevorzugt zu mindestens 99,0 Gew.-% und ganz besonders bevorzugt zu mindestens 100,0 Gew.-% addieren.

Durch das erfindungsgemäße Verfahren soll der pH-Wert der Mischung (M1), die durch Vermischen von (K1) und (K2) hergestellt wird, im Vergleich zum pH-Wert der Komponente (K1) in definierter und reproduzierbarer Weise herabgesenkt werden. Weiterhin soll auch der pH-Wert der Mischung (M2), die durch Vermischen von (M1) mit (K3) hergestellt wird, im Vergleich zur Mischung (M1) nochmals definiert und reproduzierbar herabgesenkt werden. Durch das Absenken des pH-Wertes soll die durch die (wiederholte) oxidative Färbung hervorgerufene Schädigung nicht weiter vergrößert werden. In diesem Zusammenhang hat sich herausgestellt, dass die Haarschädigung bereits signifikant vermindert werden kann, wenn der pH-Wert der Mischung (M1) um mindestens 0,2 Einheiten niedriger ist als der pH-Wert der Komponente (K1) und wenn weiterhin der pH-Wert der Mischung (M2) um mindestens 0,2 Einheiten niedriger ist als der pH-Wert der Mischung (M1).

In einer weiteren ganz besonders bevorzugten Ausführungsform ist das Verfahren zum Färben von menschlichen Haaren, dadurch gekennzeichnet, dass
- alle drei Komponenten (K1), (K2) und (K3) Wasser enthalten und
- der pH-Wert der Mischung (M1) um mindestens 0,2 Einheiten niedriger ist als der pH-Wer der Komponente (K1) und
- der pH-Wert der Mischung (M2) um mindestens 0,2 Einheiten niedriger ist als der pH-Wert der Mischung (M1).

Zur Verminderung des pH-Wertes der Farbcreme (K1) und zur Reduktion ihres Farbstoffgehalts wird der Farbverdünner (K2) mit der Farbcreme (K1) vermischt. Bei der Farbcreme (K1) handelt es sich um eine Komponente, die mindestens ein Oxidationsfarbstoffvorprodukt vom Entwicklertyp und mindestens ein Oxidationsfarbstoffvorprodukt vom Kupplertyp enthält.

Bevorzugte zusätzliche Oxidationsfarbstoffvorprodukte vom Entwickler-Typ können ausgewählt werden aus der Gruppe, die gebildet wird aus 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetra-oxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

In einer weiteren ganz besonders bevorzugten Ausführungsform ist das Verfahren zum Färben von menschlichen Haaren, dadurch gekennzeichnet, dass
- die erste Komponente (K1) mindestens ein Oxidationsfarbstoffvorprodukt aus der Gruppe aus p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)-methan, 4-Aminophenol, 4-Amino-3-methylphenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin und/oder deren physiologisch verträglichen Salzen enthält.

Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Dihydroxybenzol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.
Gemische aus zwei oder mehreren Verbindungen aus einer oder mehreren dieser Klassen sind im Rahmen dieser Ausführungsform ebenso erfindungsgemäß.

Bevorzugte Oxidationsfarbstoffvorprodukte vom Kupplertyp können ausgewählt werden aus der Gruppe aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin und/oder 7-Hydroxyindolin und deren physiologisch verträglichen Salzen.

Weiterhin kann die Komponente (K1) zusätzlich auch noch einen oder mehrere direktziehende Farbstoffe enthalten.

Der pH-Wert der Komponente (K1) ist alkalisch eingestellt. Die zur Einstellung der bevorzugten pH-Werte erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)-Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.
In einer weiteren Ausführungsform besonders bevorzugt ist ein Verfahren zum Färben von menschlichen Haaren, umfassend die folgenden Schritte in der angegebenen Reihenfolge
A) Vermischen einer ersten Komponente (K1) mit einer zweiten Komponente (K2) unter Erhalt einer ersten Mischung (M1),
B) Vermischen der Mischung (M1) mit einer dritten Komponente (K3) unter Erhalt einer zweiten Mischung (M2),
C) Applizieren der Mischung (M2) auf die Haare,
D) Einwirkenlassen der Mischung (M2) für einen Zeitraum von 30 Sekunden bis 45 Minuten,
E) Ausspülen der Mischung (M2) aus den Haaren,
   wobei
   - die erste Komponente (K1) eine Färbezubereitung ist, die in einem kosmetischen Träger mindestens ein Oxidationsfarbstoffvorprodukt vom Entwicklertyp und ein mindestens ein Oxidationsfarbstoffvorprodukt vom Kupplertyp und Ammoniak enthält,
   - die zweite Komponente (K2) ein "Farbverdünner" ist, der selbst keine Oxidationsfarbstoffvorprodukte und kein Oxidationsmittel enthält, und
   - die dritte Komponente (K3) eine Oxidationsmittelzubereitung ist, die in einem kosmetischen Träger Wasserstoffperoxid enthält.

Die durch Vermischen von (K1) und (K2) erhaltene Mischung (M1) wird erfindungsgemäß im Verfahrensschritt B) mit der Komponente (K3) vermischt. Bei der Komponente (K3) handelt es sich um eine Oxidationsmittelzubereitung welche mindestens ein Oxidationsmittel enthält. Bei dem Oxidationsmittel handelt es sich ganz besonders bevorzugt um Wasserstoffperoxid.

Die Menge an Oxidationsmittel wird der Fachmann in Abhängigkeit von der gewünschten Aufhellleistung wählen. Wenn die Ausbildung einer sehr dunklen Nuance, gewünscht wird, wird der Fachmann die Einsatzmenge an Wasserstoffperoxid entsprechend reduzieren. Soll jedoch eine leuchtende Modenuance auf dunklem Haar erzielt werden, muss das Haar gleichzeitig auch in einem signifikanten Ausmaß aufgehellt werden. In diesem Fall wird die Einsatzmenge an Wasserstoffperoxid entsprechend hoch gewählt. Die Oxidationsmittelzubereitung (K3) kann - bezogen auf das Gesamtgewicht der Komponente (K3) - 1,5 bis 12,5 Gew.-%, bevorzugt 2,5 bis 10,5 Gew.-%, weiter bevorzugt 3,0 bis 9,0 Gew.-% Wasserstoffperoxid (berechnet als 100 %iges Wasserstoffperoxid) enthalten.

In einer weiteren ganz besonders bevorzugten Ausführungsform ist das Verfahren zum Färben von menschlichen Haaren, dadurch gekennzeichnet, dass die dritte Komponente (K3)- bezogen auf das Gesamtgewicht der Komponente (K3) 1,5 bis 12,5 Gew.-%, bevorzugt 2,5 bis 10,5 Gew.-%, weiter bevorzugt 3,0 bis 9,0 Gew.-% Wasserstoffperoxid enthält.

Die Komponente (K1) und/oder die Komponente (K3) können ferner auch weitere Wirk-, Hilfs- und Zusatzstoffe enthalten, wie beispielsweise Fettbestandteile, Tenside, nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Bevorzugt werden die Komponenten (K1) und (K2) im erfindungsgemäßen Verfahren in bestimmten Mengenbereichen miteinander vermischt. Die erste Komponente (K1) und die zweite Komponente (K2) können beispielsweise in einem Mengenverhältnis von 3:1 bis 1:3, bevorzugt von 2:1 bis 2:1, miteinander vermischt werden.

Bevorzugt werden die Komponenten (K1) und (K2) im erfindungsgemäßen Verfahren in bestimmten Mengenbereichen miteinander vermischt. Die erste Mischung (M1) und die dritte Mischung (M2) können beispielsweise in einem Mengenverhältnis von 3:1 bis 1:3, bevorzugt von 2:1 bis 2:1, miteinander vermischt werden.

### Beispiel:

Werden in Schritt A) 200 g der Komponente (K1) werden mit 100 g der Komponente (K2) vermischt, so beträgt das Mengenverhältnis 2:1.

In einer weiteren ganz besonders bevorzugten Ausführungsform ist das Verfahren zum Färben von menschlichen Haaren, dadurch gekennzeichnet, dass
A) die erste Komponente (K1) und die zweite Komponente (K2) in einem Mengenverhältnis von 3:1 bis 1:3, bevorzugt von 2:1 bis 2:1, miteinander vermischt werden und
B) die Mischung (M1) und die dritte Komponente (K3) in einem Mengenverhältnis von 3:1 bis 1:3, bevorzugt von 2:1 bis 1:2, miteinander vermischt werden

Wie bereits zuvor beschrieben, soll das erfindungsgemäße Verfahren den Friseur auch in die Lage versetzen, besonders geschädigte Partien des Haares gezielt mit einem weniger schädigenden oxidativen Färbemittel zu behandeln.

In geschädigtem Haar ist die Cuticula, die Schuppenschicht des Haares, in einem mehr oder weniger starken Ausmaß zerstört. Dies führt dazu, dass auf geschädigtem Haar generell ein stärkerer Farbaufzug stattfindet. Wenn Ansatz und Spitzen mit demselben Färbemittel gefärbt werden, besteht bei stärker geschädigtem Haar daher immer die Gefahr eines ungleichmäßigen Farbergebnisses.

Es war eine zweite Aufgabe der vorliegenden Erfindung, dem Friseur ein System zur Verfügung zu stellen, mit dem er nach der Beurteilung des Schädigungsgrades des zu färbenden Haares die Farbstoffkonzentration im Haarfärbemittel einfach, gezielt, genau reproduzierbar herabsetzen kann.

Abhängig vom Schädigungsgrad der jeweiligen Haarpartie kann der Friseur somit bevorzugt wählen, ob er die Mischung (M2) in Schritt C) des Verfahrens auf die Haare im gesamten zu färbenden Bereich appliziert, oder ab der die Mischung (M2) gezielt nur auf bestimmte Haarpartien aufträgt.

In einer weiteren ganz besonders bevorzugten Ausführungsform ist das Verfahren zum Färben von menschlichen Haaren, dadurch gekennzeichnet, dass C) die Mischung (M2) auf die Haare im Ansatzbereich oder im Bereich der Haarlängen/Haarspitzen appliziert wird.

Weiterhin kann der Friseur abhängig vom Schädigungsgrad auch die Einwirkzeit der Mischung (M2) auf bestimmte Haarpartien unterschiedlich wählen.

Nach dem Auftragen auf die Haare wird die Mischung (M2) in Schritt D) für einen Zeitraum von 30 Sekunden bis 45 Minuten auf die Haare einwirken gelassen. Hierbei ist es möglich und erfindungsgemäß, die Mischung (M2) für einen bestimmten Zeitraum auf allen Partien des Haares zu belassen. In einer weiteren Ausführungsform ist es jedoch ebenfalls möglich, die Einwirkdauer für bestimmte Haarpartien verschieden zu wählen, so dass beispielsweise die Einwirkdauer im Bereich das Haaransatzes länger ist als die Einwirkdauer im Bereich der geschädigten Spitzen.

In einer weiteren ganz besonders bevorzugten Ausführungsform ist das Verfahren zum Färben von menschlichen Haaren, dadurch gekennzeichnet, dass
D1) die Mischung (M2) für einen Zeitraum von 30 Sekunden bis 45 Minuten auf die Haare im Bereich des Haaransatzes oder der Haarlängen/Haarspitzen appliziert wird und
D2) die Mischung (M2) für einen Zeitraum von 30 Sekunden bis 45 Minuten auf die Haare in dem Bereich appliziert wird, der in Schritt D1) noch nicht behandelt wurde,
wobei die Applikationsdauern der Schritte D1) und D2) sich um mindestens 5 Minuten, bevorzugt mindestens 10 Minuten unterscheiden.

Sind beispielsweise die Haare im gesamten Bereich - außer direkt am Ansatz - sehr stark geschädigt, so kann der Friseur
D1) die Mischung (M2) für einen Zeitraum von 35 bis 45 Minuten auf die Haare im Bereich des Haaransatzes applizieren und
D2) die Mischung (M2) nur für einen Zeitraum von 25 bis 30 Minuten auf die Haare im Bereich außerhalb des Ansatzes applizieren.

Sind lediglich die Spitzen stark geschädigt, so kann der Friseur
D1) die Mischung (M2) für einen Zeitraum von 15 bis 25 Minuten auf die Haare im Bereich der Haarspitzen applizieren und
D2) die Mischung (M2) nur für einen Zeitraum von 25 bis 30 Minuten auf die Haare im Bereich außerhalb der Haarspitzen applizieren.

Unter dem Haaransatz werden erfindungsgemäß die direkt an der Kopfhaut befindliche Haarpartie (die ersten 0 bis 5 cm des Haares) verstanden.

Unter dem Bereich der Haarlängen oder Haarspitzen werden - abhängig von der Haarlänge - die letzten 5 bis 10 cm des Haares verstanden).

### Beispiele

Es wurden die folgenden Formulierungen hergestellt - alle Angaben erfolgen, sofern nicht anders angegeben, in Gewichtsprozent.

### 1. Färbezubereitung (erste Komponente (K1))

| | Gew.-% |
|---|---|
| Polyacrylsäure (Ammoniumsalz) 0,5 %ige wässrige Lösung | 15,0 |
| Decyloleat | 2,1 |
| Natriumcetearylsulfat | 1,3 |
| Cetearylalkohol | 14,9 |
| Glycerylstearat | 5,4 |
| Linoleamidopropyl PG-dimoniumchlorid Phosphat | 0,05 |
| EDTA | 0,8 |
| Monoethanolamin | 0,4 |
| Ammoniak (25 %ige wässrige Lösung) | 8,0 |
| Ascorbinsäure | 0,1 |
| Natriumdithionit | 0,1 |
| L-SerinO | 0,3 |
| Polyquaternium-2 | 0,1 |
| p-Toluylendiamin, sulfat | 0,8 |
| Resorcin | 0,2 |
| m-Aminophenol | 0,04 |
| 4-Chlorresorcin | 0,2 |
| 2-Amino-4-[(2-hydroxyethyl)amino]-anisol | 0,02 |
| Wasser | ad 100 |

### 2. Farberdünner (Komponente (K2))

| | Gew.-% |
|---|---|
| Milchsäure | 4,0 |
| Paraffinium Liquidum | 50,0 |
| Cetearylalkohol | 3,4 |
| Ceteareth-20 | 5,5 |
| Glycerylstearat | 3,4 |
| Wasser | Ad 100 |
| | (pH = 1,79) |

### 3. Oxidationsmittelzuberietunq (Komponente (K3))

| | Gew.-% |
|---|---|
| Natriumbenzoat | 0,04 |
| Dipicolinsäure | 0,1 |
| Dinatriumpyrophosphat | 0,1 |
| Kaliumhydroxid | 0,1 |
| 1,2-Propandiol | 1,0 |
| Etidronsäure (1-Hydroxyehtan-1,1-diphosphonsäure) | 0,2 |
| Paraffinum Liquidum | 0,3 |
| Steartrimoniumchlorid | 0,4 |
| Cetearylalkohol | 3,4 |
| Ceteareth-20 | 1,0 |
| Wasserstoffperoxid | 6,1 |
| Wasser | ad 100 |

### 3. Anwendung

Zunächst wurde die Färbezubereitung (K1) mit dem Farbverdünner (K2) vermischt. Bei diesem Mischvorgang wurde die Mischung (M1) erhalten.

Dann wurde die Mischung (M1) mit der Oxidationsmittelzubereitung (K3) vermischt. Hierbei wurde die Mischung (M2) erhalten.

Es wurden die folgenden pH-Werte erhalten

| | (K1) + (K3) | [(K1) + (K2)] + (K3) |
|---|---|---|
| | Vergleich | Erfindung |
| pH-Wert | 10,05 | 9,78 |

## Patentansprüche

1. Verfahren zum Färben von menschlichen Haaren, umfassend die folgenden Schritte in der angegebenen Reihenfolge
A) Vermischen einer ersten Komponente (K1) mit einer zweiten Komponente (K2) unter Erhalt einer ersten Mischung (M1),
B) Vermischen der Mischung (M1) mit einer dritten Komponente (K3) unter Erhalt einer zweiten Mischung (M2),
C) Applizieren der Mischung (M2) auf die Haare,
D) Einwirkenlassen der Mischung (M2) für einen Zeitraum von 30 Sekunden bis 45 Minuten,
E) Ausspülen der Mischung (M2) aus den Haaren,
wobei
- die erste Komponente (K1) eine Färbezubereitung ist, die mindestens ein Oxidationsfarbstoffvorprodukt enthält, und
- die zweite Komponente (K2) ein Farbverdünner ist, der selbst keine Oxidationsfarbstoffvorprodukte und kein Oxidationsmittel enthält, und
- bezogen auf das Gesamtgewicht der zweiten Komponente (K2) - einen oder mehrere Fettbestandteile in einer Gesamtmenge von 5,0 bis 70,0 Gew.-% und eine oder mehrere Säuren in einer Gesamtmenge von 0,5 bis 15,0 Gew.-% enthält, und
- die dritte Komponente (K3) eine Oxidationsmittelzubereitung ist, die mindestens ein Oxidationsmittel enthält.

2. Verfahren nach Anspruch 1, **dadurch gekennzeichnet, dass**
- die zweite Komponente (K2) - bezogen auf das Gesamtgewicht der zweiten Komponente (K2) - einen oder mehrere Fettbestandteile in einer Gesamtmenge von 10,0 bis 65,0 Gew.-%, bevorzugt von 20,0 bis 60,0 Gew.-% und ganz besonders bevorzugt von 40,0 bis 60,0 Gew.-% enthält.

3. Verfahren nach einem der Ansprüche 1 bis 2, **dadurch gekennzeichnet, dass**
- die zweite Komponente (K2) - bezogen auf das Gesamtgewicht der zweiten Komponente (K2) - einen oder mehrere Kohlenwasserstoffe in einer Gesamtmenge von 20,0 bis 65,0 Gew.-%, bevorzugt von 25,0 bis 60,0 Gew.-%, weiter bevorzugt von 35,0 bis 60,0 Gew.-% und besonders bevorzugt von 40,0 bis 55,0 Gew.-% enthält.

4. Verfahren nach einem der Ansprüche 1 bis 3, **dadurch gekennzeichnet, dass**
- die zweite Komponente (K2) mindestens eine Säure aus der Gruppe aus Milchsäure, Zitronensäure, Äpfelsäure, Weinsäure, Maleinsäure, Bernsteinsäure, Oxalsäure Ascorbinsäure, Schwefelsäure, Salzsäure und/oder Phosphorsäure enthält.

5. Verfahren nach einem der Ansprüche 1 bis 4, **dadurch gekennzeichnet, dass**
- die zweite Komponente (K2) - bezogen auf das Gesamtgewicht der zweiten Komponente (K2) - eine oder mehrere Säuren in einer Gesamtmenge von 0,5 bis 8,0 Gew.-%, bevorzugt 1,5 bis 6,0 Gew.-% und ganz besonders bevorzugt von 2,5 bis 5,5 Gew.-% enthält.

6. Verfahren nach einem der Ansprüche 1 bis 5, **dadurch gekennzeichnet, dass**
- die zweite Komponente (K2) wässrig ist und einen pH-Wert von 0,5 bis 5,5, bevorzugt von 1,0 bis 4,5, weiter bevorzugt von 1,0 bis 3,5 und ganz besonders bevorzugt von 1,0 bis 2,5 besitzt.

7. Verfahren nach einem der Ansprüche 1 bis 6, **dadurch gekennzeichnet, dass**
- die zweite Komponente (K2) - bezogen auf das Gesamtgewicht der Komponente (K2) - einen Wassergehalt von 20,0 bis 70,0 Gew.-%, bevorzugt von 24,0 bis 60,0 Gew.-%, weiter bevorzugt von 24,0 bis 50,0 Gew.-% und ganz besonders bevorzugt von 28,0 bis 38,0 Gew.-% besitzt.

8. Verfahren nach einem der Ansprüche 1 bis 7, **dadurch gekennzeichnet, dass**
- die zweite Komponente (K2) ein oder mehrere nichtionische Tenside enthält.

9. Verfahren nach einem der Ansprüche 1 bis 8, **dadurch gekennzeichnet, dass**
- die zweite Komponente (K2) einen oder mehrere Fettbestandteile und eine oder mehrere Säuren und ein oder mehrere nichtionische Tenside und Wasser enthält, wobei sich - bezogen auf das Gesamtgewicht der zweiten Komponente (K2) - die Mengen aus Fettbestandteilen, Säuren, nichtionischen Tensiden und Wasser zu mindestens 95,0 Gew.-%, bevorzugt mindestens 97,0 Gew.-%, weiter bevorzugt zu mindestens 99,0 Gew.-% und ganz besonders bevorzugt zu mindestens 100,0 Gew.-% addieren.

10. Verfahren nach einem der Ansprüche 1 bis 9, **dadurch gekennzeichnet, dass**
- alle drei Komponenten (K1), (K2) und (K3) Wasser enthalten und
- der pH-Wert der Mischung (M1) um mindestens 0,2 Einheiten niedriger ist als der pH-Wer der Komponente (K1) und
- der pH-Wert der Mischung (M2) um mindestens 0,2 Einheiten niedriger ist als der pH-Wert der Mischung (M1).

11. Verfahren nach einem der Ansprüche 1 bis 10, **dadurch gekennzeichnet, dass**
- die erste Komponente (K1) mindestens ein Oxidationsfarbstoffvorprodukt aus der Gruppe aus p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, Bis-(2-hydroxy-5-aminophenyl)methan, 4-Aminophenol, 4-Amino-3-methylphenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin und/oder deren physiologisch verträglichen Salzen enthält.

12. Verfahren nach einem der Ansprüche 1 bis 11, **dadurch gekennzeichnet, dass**
- die dritte Komponente (K3) - bezogen auf das Gesamtgewicht der Komponente (K3) 1,5 bis 12,5 Gew.-%, bevorzugt 2,5 bis 10,5 Gew.-%, weiter bevorzugt 3,0 bis 9,0 Gew.-% Wasserstoffperoxid enthält.

13. Verfahren nach einem der Ansprüche 1 bis 12, **dadurch gekennzeichnet, dass**
A) die erste Komponente (K1) und die zweite Komponente (K2) in einem Mengenverhältnis von 3:1 bis 1:3, bevorzugt von 2:1 bis 2:1, miteinander vermischt werden und
B) die Mischung (M1) und die dritte Komponente (K3) in einem Mengenverhältnis von 3:1 bis 1:3, bevorzugt von 2:1 bis 1:2, miteinander vermischt werden.

14. Verfahren nach einem der Ansprüche 1 bis 13, **dadurch gekennzeichnet, dass**
C) die Mischung (M2) auf die Haare im Ansatzbereich oder im Bereich der Haarlängen/Haarspitzen appliziert wird.

15. Verfahren nach einem der Ansprüche 1 bis 14, **dadurch gekennzeichnet, dass** D1)
die Mischung (M2) für einen Zeitraum von 30 Sekunden bis 45 Minuten auf die Haare im Bereich des Haaransatzes oder der Haarlängen/Haarspitzen appliziert wird und
D2) die Mischung (M2) für einen Zeitraum von 30 Sekunden bis 45 Minuten auf die Haare in dem Bereich appliziert wird, der in Schritt D1) noch nicht behandelt wurde,
wobei die Applikationsdauern der Schritte D1) und D2 sich um mindestens 5 Minuten, bevorzugt mindestens 10 Minuten unterscheiden.

## Claims

1. A method for dyeing human hair, comprising the following steps in the order given
A) mixing a first component (K1) with a second component (K2) to obtain a first mixture (M1),
B) mixing the mixture (M1) with a third component (K3) to obtain a second mixture (M2),
C) applying the mixture (M2) to the hair,
D) allowing the mixture (M2) to act for a period of 30 seconds to 45 minutes,
E) rinsing the mixture (M2) out of the hair,
wherein
- the first component (K1) is a dye preparation which contains at least one oxidation dye precursor, and
- the second component (K2) is a dye thinner which itself contains no oxidation dye precursors and no oxidizing agent, and
- based on the total weight of the second component (K2) - contains one or more fat constituents in a total amount of 5.0 to 70.0 wt.% and one or more acids in a total amount of 0.5 to 15.0 wt.%, and
- the third component (K3) is an oxidizing agent preparation which contains at least one oxidizing agent.

2. The method according to claim 1, **characterized in that**
- the second component (K2) - based on the total weight of the second component (K2) - contains one or more fat constituents in a total amount of 10.0 to 65.0 wt.%, preferably 20.0 to 60.0 wt.%, and very particularly preferably 40.0 to 60.0 wt.%.

3. The method according to one of claims 1 to 2, **characterized in that**
- the second component (K2) - based on the total weight of the second component (K2) - contains one or more hydrocarbons in a total amount of 20.0 to 65.0 wt.%, preferably 25.0 to 60.0 wt.%, more preferably 35.0 to 60.0 wt.%, and very particularly preferably 40.0 to 55.0 wt.%.

4. The method according to one of claims 1 to 3, **characterized in that**
- the second component (K2) contains at least one acid from the group consisting of lactic acid, citric acid, malic acid, tartaric acid, maleic acid, succinic acid, oxalic acid, ascorbic acid, sulfuric acid, hydrochloric acid and/or phosphoric acid.

5. The method according to one of claims 1 to 4, **characterized in that**
- the second component (K2) - based on the total weight of the second component (K2) - contains one or more acids in a total amount of 0.5 to 8.0 wt.%, preferably 1.5 to 6.0 wt.%, and very particularly preferably 2.5 to 5.5 wt.%.

6. The method according to one of claims 1 to 5, **characterized in that**
- the second component (K2) is aqueous and has a pH of 0.5 to 5.5, preferably 1.0 to 4.5, more preferably 1.0 to 3.5, and particularly preferably 1.0 to 2.5.

7. The method according to one of claims 1 to 6, **characterized in that**
- the second component (K2) - based on the total weight of the second component (K2) - has a water content of 20.0 to 70.0 wt.%, preferably 24.0 to 60.0 wt.%, more preferably 24.0 to 50.0 wt.%, and very particularly preferably 28.0 to 38.0 wt.%.

8. The method according to one of claims 1 to 7, **characterized in that**
- the second component (K2) contains one or more non-ionic surfactants.

9. The method according to one of claims 1 to 8, **characterized in that**
- the second component (K2) contains one or more fat constituents and one or more acids and one or more non-ionic surfactants and water, the amounts of fat constituents, acids, non-ionic surfactants and water - based on the total weight of the second component (K2) - adding up to at least 95.0 wt.%, preferably at least 97.0 wt.%, more preferably at least 99.0 wt.%, and very particularly preferably at least 100.0 wt.%.

10. The method according to one of claims 1 to 9, **characterized in that**
- all three components (K1), (K2) and (K3) contain water and
- the pH of the mixture (M1) is at least 0.2 units lower than the pH of the component (K1) and
- the pH of the mixture (M2) is at least 0.2 units lower than the pH of the mixture (M1).

11. The method according to one of claims 1 to 10, **characterized in that**
- the first component (K1) contains at least one oxidation dye precursor from the group consisting of p-toluenediamine, 2-(2-hydroxyethyl)-p-phenylenediamine, N,N-bis-(2-hydroxyethyl)-p-phenylenediamine, 2-methoxymethyl-p-phenylenediamine, N-(4-amino-3-methylphenyl)-N-[3-(1H-imidazol-1-yl)propyl]amine, bis-(2-hydroxy-5-aminophenyl)methane, 4-aminophenol, 4-amino-3-methylphenol, 4,5-diamino-1-(2-hydroxyethyl)pyrazole, 2,4,5,6-tetraaminopyrimidine, 4-hydroxy-2,5,6-triaminopyrimidine, 2-hydroxy-4,5,6-triaminopyrimidine and/or the physiologically acceptable salts thereof.

12. The method according to one of claims 1 to 11, **characterized in that**
- the third component (K3) - based on the total weight of the component (K3) - contains 1.5 to 12.5 wt.%, preferably 2.5 to 10.5 wt.%, more preferably 3.0 to 9.0 wt.%, hydrogen peroxide.

13. The method according to one of claims 1 to 12, **characterized in that**
A) the first component (K1) and the second component (K2) are mixed with one another in a ratio of from 3:1 to 1: 3, preferably from 2:1 to 2:1, and
B) the mixture (M1) and the third component (K3) are mixed with one another in a ratio of from 3:1 to 1:3, preferably from 2:1 to 1:2.

14. The method according to one of claims 1 to 13, **characterized in that**
C) the mixture (M2) is applied to the hair in the roots region or in the region of the hair lengths/hair tips.

15. The method according to one of claims 1 to 14, **characterized in that** D1)
the mixture (M2) is applied to the hair in the region of the hair roots or the hair lengths/hair tips for a period of 30 seconds to 45 minutes and
D2) the mixture (M2) is applied to the hair in the region which has not yet been treated in step D1) for a period of 30 seconds to 45 minutes,
the application durations of steps D1) and D2 differing by at least 5 minutes, preferably at least 10 minutes.

## Revendications

1. Procédé de coloration de cheveux humains, comprenant les étapes suivantes dans l'ordre indiqué
A) mélange d'un premier constituant (K1) avec un deuxième constituant (K2) pour obtenir un premier mélange (M1),
B) mélange du mélange (M1) avec un troisième constituant (K3) pour obtenir un second mélange (M2),
C) application du mélange (M2) sur les cheveux,
D) pose du mélange (M2) pendant une durée de 30 secondes à 45 minutes,
E) élimination par rinçage du mélange (M2) présents sur les cheveux,
- le premier constituant (K1) étant une préparation colorante qui contient au moins un précurseur de colorant d'oxydation, et
- le deuxième constituant (K2) étant un diluant de colorant qui lui-même ne contient aucun précurseur de colorant d'oxydation ni agent oxydant, et
- par rapport au poids total du deuxième constituant (K2) - un ou plusieurs composants gras en une quantité totale comprise entre 5,0 et 70,0 % en poids et un ou plusieurs acides en une quantité totale comprise entre 0,5 et 15,0 % en poids, et
- le troisième constituant (K3) étant une préparation d'agent oxydant qui contient au moins un agent oxydant.

2. Procédé selon la revendication 1, **caractérisé en ce que**
- le deuxième constituant (K2) contient - par rapport au poids total du deuxième constituant (K2) - un ou plusieurs composants gras en une quantité totale comprise entre 10,0 et 65,0 % en poids, de préférence entre 20,0 et 60,0 % en poids et de manière tout particulièrement préférée entre 40,0 et 60,0 % en poids.

3. Procédé selon l'une des revendications 1 à 2, **caractérisé en ce que**
- le deuxième constituant (K2) contient - par rapport au poids total du deuxième constituant (K2) - un ou plusieurs hydrocarbures en une quantité totale comprise entre 20,0 et 65,0 % en poids, de préférence entre 25,0 et 60,0 % en poids, plus préférablement entre 35,0 et 60,0 % en poids et de manière particulièrement préférée entre 40,0 et 55,0 % en poids.

4. Procédé selon l'une des revendications 1 à 3, **caractérisé en ce que**
- le deuxième constituant (K2) contient au moins un acide du groupe constitué par l'acide lactique, l'acide citrique, l'acide malique, l'acide tartrique, l'acide maléique, l'acide succinique, l'acide oxalique, l'acide ascorbique, l'acide sulfurique, l'acide chlorhydrique et/ou l'acide phosphorique.

5. Procédé selon l'une des revendications 1 à 4, **caractérisé en ce que**
- le deuxième constituant (K2) contient - par rapport au poids total du deuxième constituant (K2) - un ou plusieurs acides en une quantité totale comprise entre 0,5 et 8,0 % en poids, de préférence entre 1,5 et 6,0 % en poids et de manière tout particulièrement préférée entre 2,5 et 5,5 % en poids.

6. Procédé selon l'une des revendications 1 à 5, **caractérisé en ce que**
- le deuxième constituant (K2) est aqueux et présente un pH compris entre 0,5 et 5,5, de préférence entre 1,0 et 4,5, plus préférablement entre 1,0 et 3,5 et de manière tout particulièrement préférée entre 1,0 et 2,5.

7. Procédé selon l'une des revendications 1 à 6, **caractérisé en ce que**
- le deuxième constituant (K2) contient - par rapport au poids total du constituant (K2) - une teneur en eau comprise entre 20,0 et 70,0 % en poids, de préférence entre 24,0 et 60,0 % en poids, plus préférablement entre 24,0 et 50,0 % en poids et de manière tout particulièrement préférée entre 28,0 et 38,0 % en poids.

8. Procédé selon l'une des revendications 1 à 7, **caractérisé en ce que**
- le deuxième constituant (K2) contient un ou plusieurs tensioactifs non ioniques.

9. Procédé selon l'une des revendications 1 à 8, **caractérisé en ce que**
- le deuxième constituant (K2) contient un ou plusieurs composants gras, un ou plusieurs acides, un ou plusieurs tensioactifs non ioniques et de l'eau, la somme des quantités de composants gras, d'acides, de tensioactifs non ioniques et d'eau représentant, par rapport au poids total du deuxième constituant (K2), au moins 95,0 % en poids, de préférence au moins 97,0 % en poids, plus préférablement au moins 99,0 % en poids et de manière tout particulièrement préférée au moins 100,0 % en poids.

10. Procédé selon l'une des revendications 1 à 9, **caractérisé en ce que**
- les constituants (K1), (K2) et (K3) contiennent tous trois de l'eau, et
- le pH du mélange (M1) est inférieur d'au moins 0,2 unité au pH du constituant (K1), et
- le pH du mélange (M2) est inférieur d'au moins 0,2 unité au pH du mélange (M1).

11. Procédé selon l'une des revendications 1 à 10, **caractérisé en ce que**
- le premier constituant (K1) contient au moins un précurseur de colorant d'oxydation du groupe constitué par la p-toluènediamine, la 2-(2-hydroxyéthyl)-p-phénylènediamine, la N,N-bis-(2-hydroxyéthyl)-p-phénylènediamine, la 2-méthoxyméthyl-p-phénylènediamine, la N-(4-amino-3-méthylphényl)-N-[3-(1H-imidazol-1-yl) propyl]amine, le bis-(2-hydroxy-5-aminophényl)-méthane, le 4-aminophénol, le 4-amino-3-méthylphénol, le 4,5-diamino-1-(2-hydroxyéthyl)-pyrazole, la 2,4,5,6-tétraaminopyrimidine, la 4-hydroxy-2,5,6-triaminopyrimidine, la 2-hydroxy-4,5,6-triaminopyrimidine et/ou leurs sels physiologiquement tolérables.

12. Procédé selon l'une des revendications 1 à 11, **caractérisé en ce que**
- le troisième constituant (K3) contient - par rapport au poids total du constituant (K3) - entre 1,5 et 12,5 % en poids, de préférence entre 2,5 et 10,5 % en poids, plus préférablement entre 3,0 et 9,0 % en poids de peroxyde d'hydrogène.

13. Procédé selon l'une des revendications 1 à 12, **caractérisé en ce que**
A) le premier constituant (K1) et le deuxième constituant (K2) sont mélangés l'un avec l'autre dans un rapport en poids compris entre 3:1 et 1:3, de préférence entre 2:1 et 2:1, et
B) le mélange (M1) et le troisième constituant (K3) sont mélangés l'un avec l'autre dans un rapport en poids compris entre 3:1 et 1:3, de préférence entre 2:1 et 1:2.

14. Procédé selon l'une des revendications 1 à 13, **caractérisé en ce que**
C) le mélange (M2) est appliqué sur les cheveux dans la zone des racines ou dans la zone des longueurs/pointes des cheveux.

15. Procédé selon l'une des revendications 1 à 14, **caractérisé en ce que** D1)
le mélange (M2) est appliqué sur les cheveux dans la zone des racines des cheveux ou sur les longueurs/pointes des cheveux pendant une durée de 30 secondes à 45 minutes, et
D2) le mélange (M2) est appliqué sur les cheveux dans la zone qui n'a pas encore été traitée à l'étape D1) pendant une durée de 30 secondes à 45 minutes,
la durée d'application des étapes D1) et D2) différant d'au moins 5 minutes, de préférence d'au moins 10 minutes.
